## Europäisches Patentamt

## European Patent Office

## Office européen des brevets

(19)

(11) Veröffentlichungsnummer : **0 076 896**
**B1**

(12) **EUROPÄISCHE PATENTSCHRIFT**

(45) Veröffentlichungstag der Patentschrift :
26.06.85

(21) Anmeldenummer : 82106833.5

(22) Anmeldetag : 29.07.82

(51) Int. Cl.⁴ : **A 61 M 25/02**, A 61 F 13/00,
A 61 F 13/02

(54) Medizinischer Verband zur Fixierung einer Sonde.

(30) Priorität : 29.07.81 DE 3129931

(43) Veröffentlichungstag der Anmeldung :
20.04.83 Patentblatt 83/16

(45) Bekanntmachung des Hinweises auf die Patenterteilung : 26.06.85 Patentblatt 85/26

(84) Benannte Vertragsstaaten :
AT BE CH DE FR GB IT LI LU NL SE

(56) Entgegenhaltungen :
US-A- 3 568 679
US-A- 3 683 911
Udo Bauer: Verpackung; 1. Auflage, 1981, Vogel-
Verlag Würzburg; S. 334-335, Abschnitt 8.2.4

(73) Patentinhaber : **Institut für angewandte Mikroskopie**
**Willi Fischer**
**Königin-Luise-Strasse 73**
**D-1000 Berlin 33 (DE)**

(72) Erfinder : **Fischer, Willi**
**Königin-Luise-Strasse 73**
**D-1000 Berlin 33 (DE)**

(74) Vertreter : **von Samson-Himmelstjerna, Friedrich R.**
**Donaustrasse 24**
**D-8000 München 80 (DE)**

EP 0 076 896 B1

## Beschreibung

Die Erfindung bezieht sich auf einen medizinischen Verband zur Fixierung einer durch die Haut eines Lebewesens in dessen Körper eingeführten Sonde. Der medizinische Verband weist hierbei einen an der Haut und am aus der Haut herausragenden Teil der Sonde gleichzeitig befestigbaren Verbandsabschnitt auf. Der Verbandsabschnitt ist mit einem zur Anlage an der Haut ausgelegten Flächengebilde und einem mit dem Flächengebilde verbundenen, von diesem stutzenartig vorstehenden und rohrförmig ausgebildeten Fortsatz bestückt. Die Innenwandung des Fortsatzes ist hierbei als Anlagefläche für den aus der Haut herausragenden Teil der Sonde ausgeformt. Zum Zwecke einer manuellen Erweiterung des Fortsatzinnenraumes beim Einlegen oder Entfernen der Sonde in den oder aus dem Fortzsatz, weist der Fortsatz einen im wesentlichen achsparallel geführten und sich über seine gesamte Länge erstreckenden Schlitz auf.

Der vorstehend gebrauchte Ausdruck « Sonde » dient als Sammelbegriff für Nadeln, Kanülen, Katheder oder dergleichen. Die Sonde kann hierbei zur Zuführung oder Abführung von Substanzen in oder aus dem Körper dienen ; ebenso zur Ableitung von Informationen über aus dem Köperinnern ablaufende Funktionen. Im letzteren Fall muß die Sonde nicht unbedingt hohl sein.

Ein medizinischer Verband zur Fixierung einer Sonde ist beispielsweise als ein mittels eines Heftpflasterstreifens realisierter « V-Verband » bekannt. Bei dem bekannten « V-Verband » wird der Heftpflasterstreifen nach Art eines Schals einmal um den aus der Haut herausragenden Teil der Sonde geschlagen, wobei sich der Heftpflasterstreifen auf der der Haut gegenüberliegenden Seite der Sonde überkreuzt und von dort' weitergeführt und an die Haut geklebt wird. Der bekannte Verband hat den Vorteil eines bequemen Anbringens. Auch ist er in der Regel medizinisch unbedenklich, insbesondere hautverträglich. Er hat jedoch den Nachteil, daß er eine vergleichsweise dürftige Fixierung bzw. Festlegung der Sonde im Körper des Lebewesens sicherstellt. Dies hat nicht nur die Gefahr einer Schmerzverursachung des Lebenwesens bei Bewegung der Sonde zur Folge, sondern darüberhinaus auch die erheblich schwerwiegendere Gefahr einer unmittelbaren Verletzung, insbesondere einer Venenreizung oder noch schlimmer eines Venendurchstoßes und die damit einhergehende erhöhte Gefahr einer Trombosebildung. Hinzu kommt, daß eine wiederholte Schmerzverursachung bei dem mit der Sonde behafteten Lebewesen zu Unruheerscheinungen und daraus folgend zu einer erhöhten Gefahr einer Bewegung der Sonde führt. Schließlich besteht auch bei dem bekannten Verband die Gefahr, daß die Sonde abknickt, insbesondere nachdem die Mandrinnadel entfernt ist. Nicht nur die Bildung von Tromben, sondern darüberhinaus auch ein Abknicken der Sonde kann zu verheerenden Schädigungen-bis hin zum Eintritt des Todes-des mit der Sonde behafteten Lebewesens führen. Gerade Katheter, die über die Vena subclavia und Vena jugularis interna eingeführt werden, sind dafür bekannt, daß sie sich leicht verschieben und gegebenenfalls knicken. um ein Verschieben der Katheter zu vermeiden, näht man dieselben daher häufig durch eine einzelne starke Seitennaht an der Haut an. Diese Maßnahme stellt zwar eine sichere Fixierung der Sonde am Lebewesen dar. Sie ist aber wenig schonend. Ähnliche Probleme bestehen auch bei Kathetern, die in der Periduralanästhesie, sowie der Peridurographie zur Darstellung des Epiduralraumes durch Kontrastmittel verwendet werden.

Anstelle des oder zusätzlich zum bekannten « V-Verband » aus Heftpflastern wird bisweilen nach Einführung der Sonde durch den Arzt, insbesondere bei längerer Verweildauer, ein Verband angelegt. Zwar findet hierdurch eine gewisse zusätzliche, dennoch aber unzureichende Fixierung der Sonde statt. Hinzu kommt, daß das Anlegen des Verbandes zeitraubend, häufig schmerzhaft, zumindest aber wegen der beim Wicklen des Verbandes wiederkehrenden Berührungen zwischen Verband und Sonde beunruhigend für den Patienten ist — mit der Folge, daß sich auch der Patient bewegt und eine schmerzhafte Berührung zwischen Verband und Sonde erst recht eintritt. Im übrigen weist dieser bekannte Verband im wesentlichen die gleichen Nachteile wie der bekannte « V-Verband » auf, wenngleich möglicherweise in abgeschwächter Form.

Der eingangsgenannte gattungsmäße medizinische Verband ist aus der US-A-3 683 911 (Mc CORMICK) bekannt. Dieser bekannte medizinische Verband gewährleistet eine ausgezeichnete Fixierung einer in den Körper eines Lebewesens eingeführten Sonde an der Haut des Lebewesens. Einhergend mit der erhöhten Fixierung der Sonde ergibt sich eine Verringerung der Infektionsgefahr, die beispielsweise durch Reizugen der Vene und/oder einer Verschlechterung des Hautschlusses an der Punktionsstelle infolge erhöhter Beweglichkeit beding ist. Insbesondere wurden beim bekannten Verband lebensbedrohende Gefahren infolge Trombosen oder Unterbrechungen der Zu- oder Abfuhr von Substanzen in oder aus dem Körper beseitigt. Die bekannte Ausbildung des Fortsatzes dient nicht nur in besonderem Maße der guten Fixierung der Sonde. Sie bewirkt auch eine gegen pathogene Erreger undurchlässige Abschirmung der Sonde. Der im wesentlichen achsparallel geführte und sich über die gesamte Fortsatzlänge erstreckende Schlitz erleichtert in erheblichem Maße ein rasches Einlegen der Sonde in den Fortsatz. Beim bekannten medizinischen Verband ist jedoch die als Anlagefläche für die Sonde dienende Innenwand des Fortsatzes mit einer Haftschicht beschichtet, die eine nachträgliche Trennung von Sonde und medizinischem Verband erheblich erschwert. Zumindest sind hierbei Zerr- und Reißbewegungen nicht auszuschließen. Derartige Zerr- und Reißbewegungen können aber zu einem Abknicken der Sonde — falls diese eine Katheter ist — oder zu Venenreizungen oder gar einem Venendurchstich — falls die Sonde eine Nadel ist — führen.

Die Erfindung befaßt sich mit dem Problem, den aus der US-A-3 683 911 bekannten medizinischen Verband so auszubilden, daß er schnell, gefahrlos, einfach und vor allem schmerzfrei am Patienten ausgewechselt werden kann.

Diese Aufgabe wird dadurch gelöst, daß — nicht die Innenwandung der Fortsatzes, sondern — die einander gegenüberliegenden Flächen des im Fortsatz geführten Schlitzes eine selbsthaftende Schicht aufweisen und der Fortsatz — zum Zwecke eines manuellen Lösens der Haftverbindung der Flächen des Schlitzes — mit einem im Innern des Fortsatzes, längs der Flächen des Schlitzes geführten und aus dem Fortsatz herausragenden Trennfaden bestückt ist.

Diese gemeinsame Verwendung selbsthaftender Schichten mit einem Trennfaden zur Lösung der selbsthaften Schichten voneinander ermöglicht ein leichtes und weiches — und damit ein patienten-schonendes Öffnen des Schlitzes im Fortsatz. Mit dem Trennfaden wird hierbei nicht — wie bei sonst üblichen Reißfäden — das einen Gegenstand umgebende Material zerstört. Es werden lediglich zwei aneinander haftende Flächen zerstörungsfrei von einander getrennt.

Grundsätzlich kann der Fortsatz in Folge der Eigensteifigkeit des verwendeten Material frei vorstehen. Vorzugsweise ist jedoch der Fortsatz über eine Verbindungsrippe mit dem Flächengebilde verbunden. Hierdurch wird die Lagestabilität des Fortsatzes und damit der Sonde erhöht (Anspruch 2).

Bevorzugt sind die Flächen des Schlitzes als Laschen ausgebildet. Hierdurch können größere Flächen mit einer Haftschicht beschichtet werden. Diese Maßnahme bietet eine erhöhte Festigkeit und Fremdstoffundurchlässigkeit des Fortsatzes. Gleichzeitig erleichtern die Laschen die Handhabbarkeit des medizinischen Verbandes, da sie auch als Greiflaschen verwendbar sind (Anspruch 3).

Gemäß einem weiteren bevorzugten Ausführungsbeispiel ist der Fußbereich des Fortsatzes allseitig vom Flächengebilde umgeben, wobei das Flächengebilde im Fußbereich eine Durchtrittsöffnung aufweist. Diese, an sich aus der US-A-3 683-911 bekannte Maßnahme hat nicht nur den Vorteil einer großflächigen Abdeckung des Applikationsortes und damit einer Verringerung der Infektionsgefahr. Hinzu kommt auch eine großflächige Ableitung von gegebenenfalls auf den Fortsatz oder die Sonde ausgeübten Druck- und/oder Zugkräften (Anspruch 4).

Eine besonders bequeme Handhabbarkeit des medizinischen Verbandes wird — wie an sich aus der genannten US-A-3-683-911 bekannt — dadurch erreicht, daß das Flächengebilde wenigstens eine vom Rand der Durchtrittsöffnung ausgehende, wenigstens bis in den Bereich der äußeren Umgrenzungskante des Flächengebildes führende freie Kante aufweist, die in der selben Ebene liegt wie die Achse des Fortsatzes ; ferner dadurch, daß die Innenkante des Schlitzes und die vorgenannte freie Kante des Flächengebildes jeweils im wesentlichen im selben Punkt des äußeren Randes der Durchgangsöffnung enden. Diese Maßnahme ermöglicht es, den medizinischen Verband längs der Haut unter die Sonde zu schieben (Anspruch 5).

Eine Erleichterung der Handhabbarkeit sowie eine Erhöhung der Abdeckung des Applikationsortes wird gemäß einem weiteren bevorzugten Ausführungsbeispiel dadurch erreicht, daß das Flächengebilde eine seine freie Kante überlappende Lasche aufweist (Anspruch 6).

Gemäß einem weiteren bevorzugten Ausführungsbeispiel besteht der Fortsatz aus dem selben Material wie das Flächengebilde, insbesondere einem für Röntgenstrahlen, sichtbares Licht und/oder Ultraschallwellen durchlässigen Material, vorzugsweise, einem elastischen Material, beispielsweise Silikon-Kautschuk, Latex-Kautschuk, Polyurethan oder Polytetrafluoräthylen. Die Verwendung eines elastischen Materials gewährleistet, daß sich das Flächengebilde bequem unterschiedlichen Körperpartien anpassen kann.

Die Verwendung von Material, das für Röntgenstrahlen, sichtbares Licht oder Ultraschallwellen durchlässig ist, ermöglichtein frühzeitiges Erkennen unerwünschter Veränderungen, beispielsweise Entzündungen, Hautallergien oder dergleichen im Bereich der Punktionsstelle. Je früher derartige Veränderungen erkannt werden, umso eher ist die Möglichkeit gegeben, derartige Veränderungen mit vergleichsweise schonenden Eingriffen zu behandeln (Ansprüche 7 bis 9).

Wie aus der genannten US-A-3 683 911 an sich bekannt, weist auch eine bevorzugte Ausführungsform des erfindungsgemäßen medizinischen Verbandes eine an der Haut des Lebewesens selbsthaftende Haftschicht auf der Unterseite des Flächengebildes auf. Diese Maßnahme dient einer raschen und bequemen Applikation des medizinischen Verbandes (Anspruch 10).

Aus der genannten US-A-3 683 911 ist es an sich bekannt, diese Haftschicht antibakteriell auszugestalten. Gamäß einer weiteren bevorzugten Ausführungsform der Erfindung ist jedoch das Flächengebilde auf seiner der Haut zugewandten Fläche mit einem längs einer geschlossenen Bahn um die Durchtrittsöffnung geführten Vlies versehen, das mit Antibiotika, entzündungshemmende Pharmaka oder einer Mischung daraus imprägniert ist.

Nachstehend wird die Erfindung anhand von Ausführungsbeispielen unter Bezugnahme auf die beigefügte schematische Zeichnung noch näher erläutert.

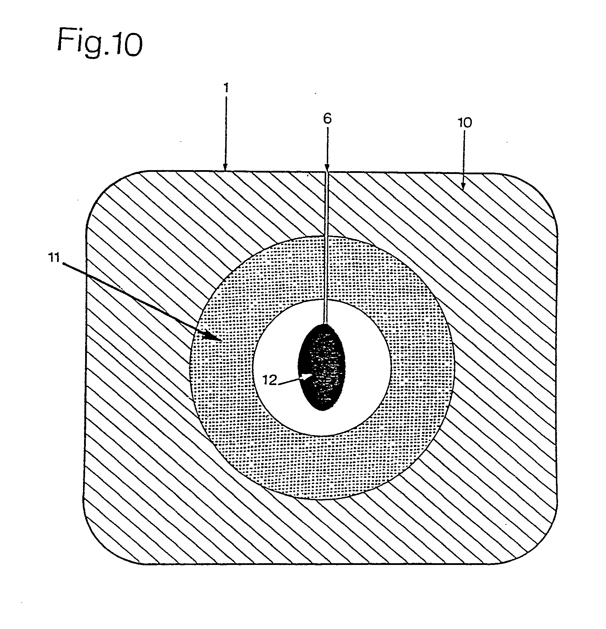

Die Teilanmeldung Nr. 84 101 298 2, Veröffentlichungsnummer 0 121 679, enthält abhängige Ansprüche und eine Zeichnung, die Merkmale von abhängigen Ansprüchen und des Ausführungsbeispieles gemäß der Figur 10 der vorliegenden Anmeldung betreffen.

In der Zeichnung zeigen :

Figur 1a bis 3a Querschnitte durch Ausführungsbeispiele in geöffnetem Zustand

Figuren 1b bis 3b die in den Figuren 1a bis 3a dargestellten Ausführungsbeispiele, jedoch in geschlossenem Zustand ;

Figuren 4 bis 7 Querschnitte durch den Fortsatz in verschiedenen Stellungen bzw. während verschiedener Arbeitsstufen ;

Figuren 8a und 8b Ansichten eines weiteren Ausführungsbeispiels in geöffnetem (Figur 8a) und geschlossenem (Figur 8b) Zustand ;

Figuren 9a und 9b Ansichten eines weiteren Ausführungsbeispiels in geöffnetem (Figur 9a) und geschlossenem (Figur 9b) Zustand ; und

Figur 10 ein weiteres Ausführungsbeispiel von unten gesehen.

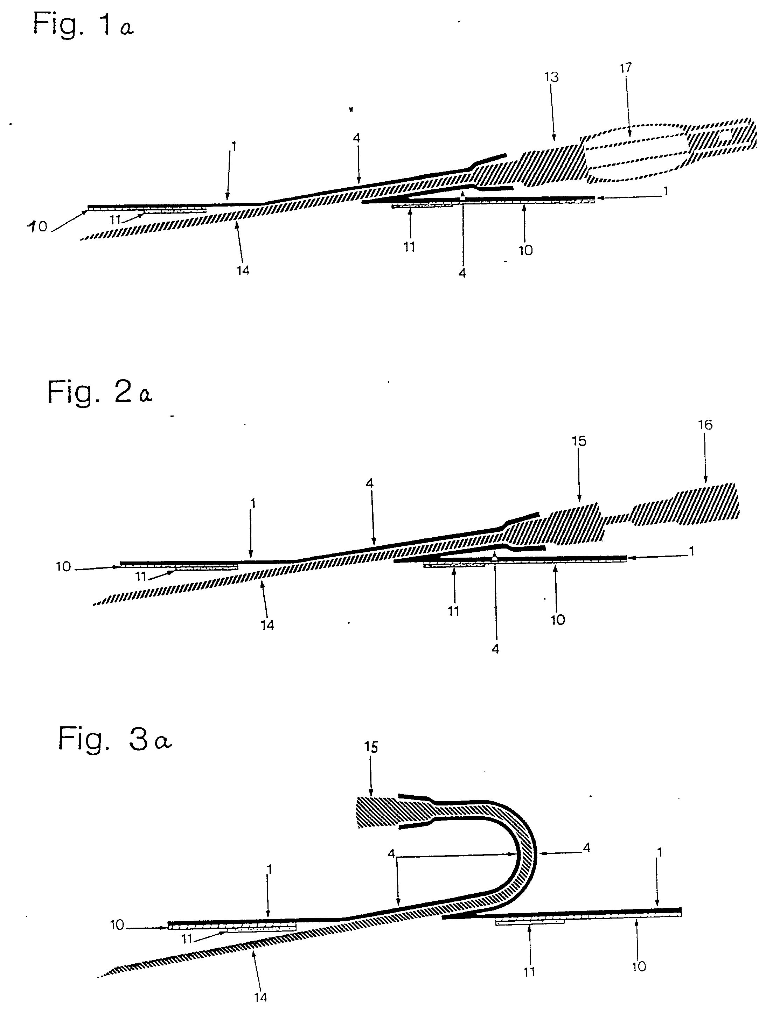

Die Figuren 1a bis 3b zeigen Querschnitte durch Ausführungsbeispiele des Verbandes nebst den von diesen zu haltenden Sonden, insbesondere Kanülen 14. In diesen Figuren sind zwar wesentliche Merkmale der Erfindung nicht dargestellt. Aus ihnen ist jedoch besonders deutlich die der Lage des eingeführten Katheters oder der Kanüle 14 angepaßte Schräglage eines noch zu erläuternden Fortsatzes 4 ersichtlich ; darüberhinaus die paßgerechte Anpassung des freien Endes des Fortsatzes 4 an jeweils handelsübliche Anschlußkonusse, Kanülenansätze und Fixierkonusse 13, 15 16.

Die Figuren 1a und 1b zeigen die Schräglage der Kanüle 14 oder eines Katheters mit Anschlußkonus 13 und Blutkammer 17.

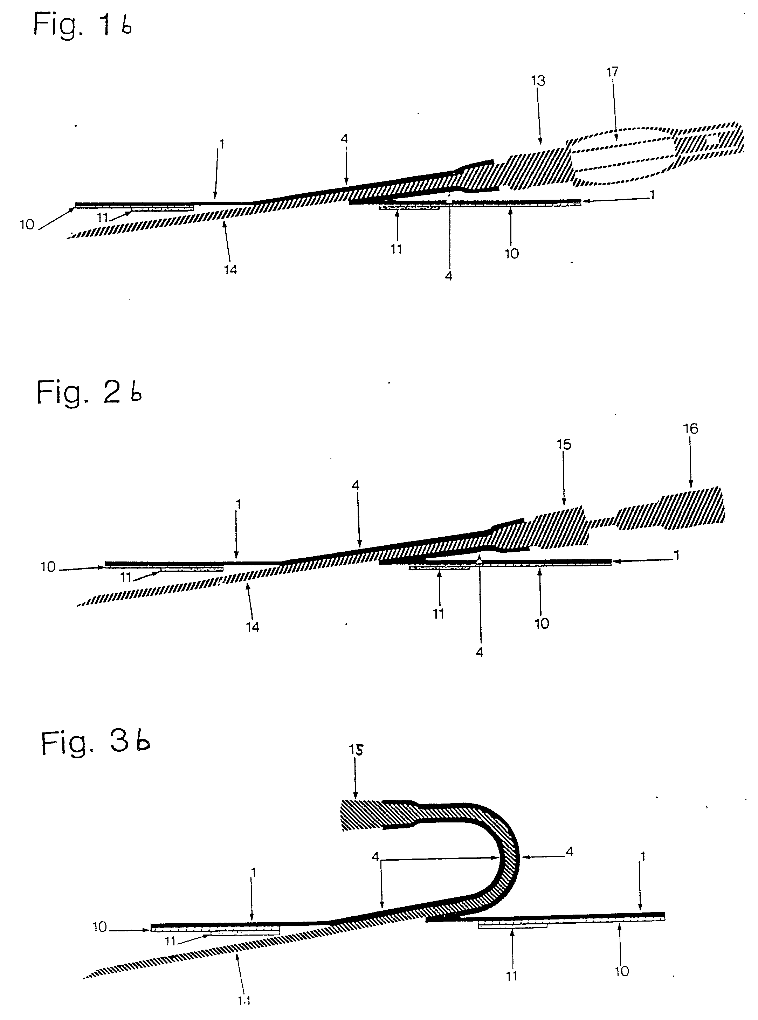

Die Figuren 2a und 2b zeigen die Schräglage einer weiteren Sonde mit einem anders ausgeformten Anschlußkonus 15 und durchgeführtem Katheter mit Anschlußkonus 16.

Die Figuren 3a und 3b zeigen die Schräglage eines eingeführten Katheters in gebogener Version.

Der gezeigte Verband dient der Festlegung einer in die Vene eines Lebewesens eingeführten Kanüle 14. Hierzu weist der Verband ein elastisches Flächengebilde 1 auf, das auf seiner Unterseite mit einer auf der Haut des Lebewesens haftenden Haftschicht 10 beschichtet ist. Die Größe des Flächengebildes 1 ist so bemessen, daß es mittels der Haftschicht 10 einen ausreichenden Halt auf der Haut findet. Vom Flächengebilde 1 erstreckt sich ein Fortsatz 4 in spitzem Winkel nach oben. Dessen Innenfläche ist der Außenfläche der Kanüle 14, einschließlich deren Anschlußkonus 15 angepaßt. Die Innenfläche des Fortsatzes 4 dient demnach als Anlagefläche 4″ für den aus der Haut herausragenden Teil der Kanüle 14. Der Winkel zwischen dem Flächengebilde 1 und der Anlagefläche 4″ des Fortsatzes 4 ist so bemessen, daß eine unter üblichem Winkel in die Vene eingeführte Kanüle 14 satt auf der Anlagefläche 4″ aufliegt. Der Fortsatz 4 und das Flächengebilde 1 bestehen im dargestellten Ausführungsbeispiel aus dem gleichen Material. Zur starren Fixierung des Fortsatzes 4 am Flächengebilde 1 kann zusätzlich noch ein Verbindungsrippe zwischen dem Flächengebilde 1 und der Unterseite des Fortsatzes 4 vorgesehen sein. Das Flächengebilde 1, der Fortsatz 4 und die Verbindungsrippe können beispielsweise aus einem einstückig gegossenen Kunststoff bestehen.

Die in den Figuren 1a bis 3b und 8a bis 10 dargestellten Ausführungsbeispiele weisen eine elastische Folie als Flächengebilde 1 auf. Je nach Bedarf ist die Folie dem Applikationsort angepaßt, d. h. sie kann rund, oval, dreieckig oder viereckig geschnitten sein. Die Folie überdeckt hierbei den gesamten Applikationsort. Um auch nach Anbringung des Verbandes den gesamten Applikationsort gut beobachten, insbesondere unerwünschte Veränderungen wie Entzündungen, Trombosebildungen oder dergleichen bequem beobachten zu können, ist die Folie durchsichtig, insbesondere auch röntgenstrahlendurchlässig. Die Röntgenstrahlendurchlässigkeit ermöglicht darüberhinaus auch eine transvenöse Extraktion der Tromben usw. durch Schlingen. Bevorzugt besteht die Folie aus Sililon-Kautschuk, Latex-Kautschuk, Polyurethan, Polytetrafluoräthylen oder ähnlichen Werkstoffen. Nur am Rande sei erwähnt, daß der für die Folie verwendete Werkstoff zur Vermeidung von Intoxikationen keine Weichmacher enthält.

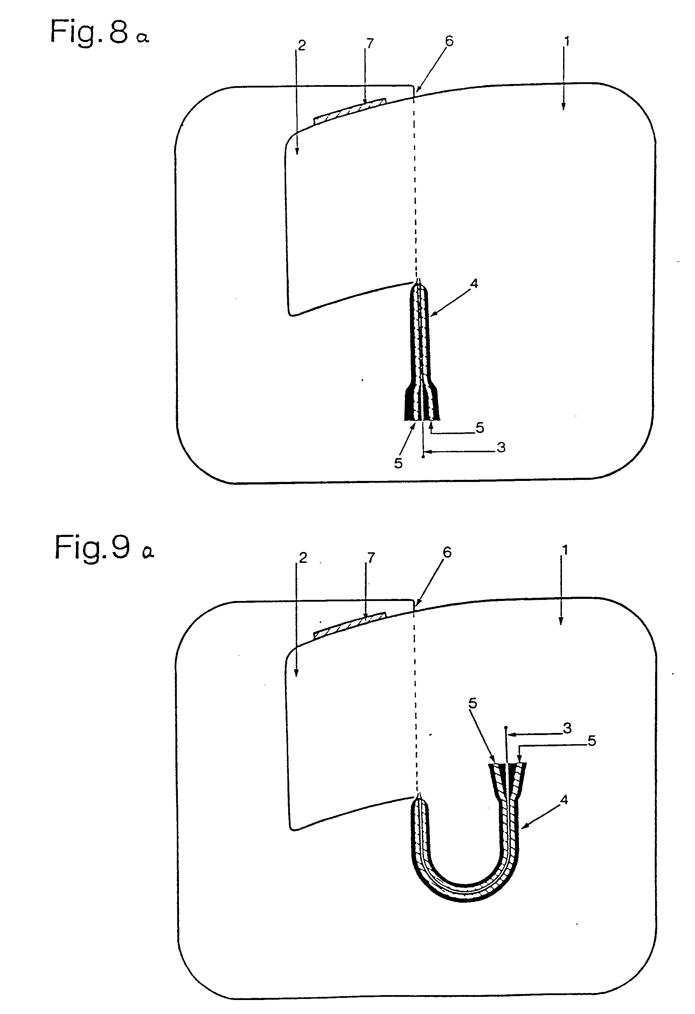

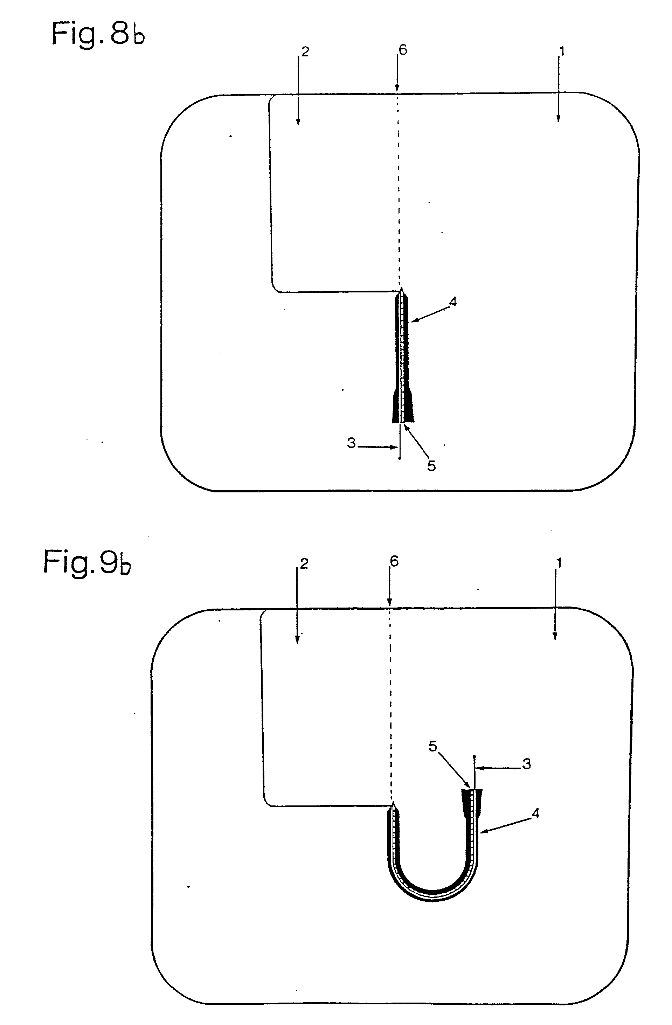

Gemäß Figur 10 weist die Folie, d. h. das Flächengebilde 1, eine mittig angeordnete ovale Durchgangsöffnung 12 für den Durchtritt der Sonde, insbesondere also der Kanüle 14, auf. Infolge eines in Richtung der großen Längsachse der ovalen Durchgangsöffnung 12 von der Außenkante des Flächengebildes 1 bis hin zum äußeren Rand der Durchgangsöffnung 12 geführten Einschnittes ist die Folie mit wenigstens einer längs der Schnittlinie laufenden freien Kante 6 versehen. Gemäß den Fig. 8a bis 9b geht der dieser freien Kante 6 gegenüberliegende Abschnitt der Folie in eine die freie Kante 6 überlappende Lasche 2 über. Durch Anheben der Lasche 2 entsteht ein zur freien Kante 6 der Folie im wesentlichen parallel verlaufender und bis zur ovalen Durchgangsöffnung 12 führender Schlitz, der ein späteres Unterschieben des Verbandes unter den aus der Haut herausragenden Teil der Sonde 14 erleichtert. Zur späteren Befestigung der Lasche 2 an der Folie ist die Lasche auf ihrer Unterseite mit einer — vorzugsweise streifenförmigen — Haftschicht 7 ausgestattet. Die Lasche 2 hat hierbei im wesentlichen den Zweck einer vollständigen Abdeckung des Applikationsortes sowie einer Erleichterung der Ablösung der Folie von der Haut. Dem letztgenannten Zweck dient auch die streifenförmige Ausbildung der Haftschicht 7.

Ein gegenüber den in den Figuren 8a bis 9b abgewandeltes Ausführungsbeispiel weist keine Lasche 2 auf. In diesem Fall wird der von der freien Kante 6 der Folie bis hin zum äußeren Rand der Durchgangsöffnung 12 führende Schlitz von zwei freien Kanten 6 begrenzt (siehe z. B. Figur 10). Eine vollständige Abdeckung des Applikationsortes ist hierbei dadurch erreichbar, daß die beiden freien Kanten 6 des Flächengebildes 1 in Wirkstellung bündig aneinander grenzen.

Bei den in den Figuren 1a bis 3b sowie 8a bis 10 dargestellten Ausführungsbeispielen setzt sich der Fortsatz 4 vom äußeren Rand der Durchgangsöffnung 12 des Flächengebildes 1 fort. Er ist rohrförmig ausgebildet und steht stutzenartig vor. In den Figuren 1a bis 2b und 8a, b ist der Fortsatz geradlinig, in den Figuren 3a, b und 9a, b gekrümmt ausgebildet. Die letztgenannten Ausführungsbeispiele dienen insbesondere der Halterung von Kathetern. Hierbei zeigen die Figuren 1a, 2a, 3a, 8a und 9a jeweils den geschlossenen Zustand des Verbandes — mit und ohne die Sonde — und die Figuren 1b, 2b, 3b, 8b und 9b jeweils den geschlossenen Zustand des Verbandes — wiederum mit und ohne Sonde.

Gemäß einem bevorzugten Ausführungsbeispiel ist der als Schlauch ausgebildete Fortsatz 4 einstückig mit dem Flächengebilde 1 verbunden und besteht bevorzugt aus dem gleichen Material wie dieses.

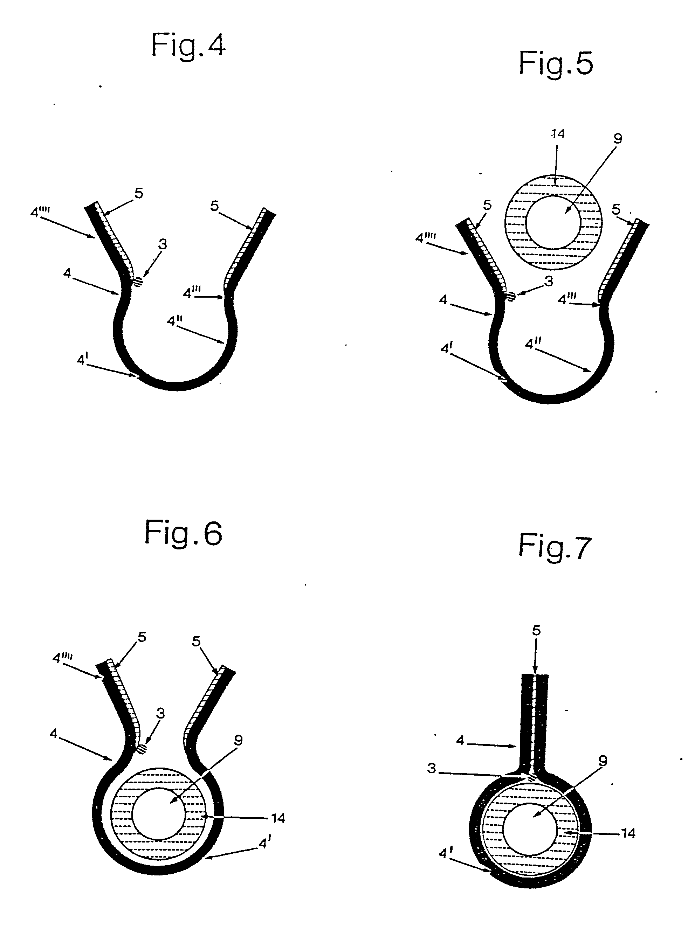

Die Figuren 4 bis 7 zeigen den rohrartig ausgebildeten Fortsatz 4 im Querschnitt während unterschiedlicher Arbeitsstellungen, wobei die Figur 4 die offene und die Figur 7 die geschlossene Arbeitsstellung wiedergeben.

Der Fortsatz 4 besteht aus einem in seiner Längsrichtung aufklappbaren Grundkörper 4' bzw. zwei diesen Grundkörper bildenden auseinanderschwenkbaren und wieder zusammenführbaren und miteinander verbundenen Fortsatzhälften. An jede Fortsatzhälfte schließt sich jeweils eine Lasche 4'''' an. Die Innenfläche 4'' des Grundkörpers 4' ist hierbei so ausgebildet, daß sie in geschlossenem Zustand des Fortzatzes 4, also in Wirkstellung, paßgerecht mit den Außenmaßen handelsüblicher Kanülen, Katheter, Nadeln oder dergleichen, einschließlich deren Anschlußkonusse 13, 15, 16 übereinstimmt. Die Laschen 4'''' sind zumindest im Bereich ihres Überganges 4''' zum Grundkörper 4 mit einer selbst haftenden Schicht, z. B. einem Haftstreifen 5 beschichtet. Vorzugsweise überdeckt der Haftstreifen 5 die gesamte Fläche der Laschen 4''''. In diesem Fall ist wegen der großen Haftfläche eine besonders sichere Fixierung der in den Fortsatz 4 eingeführten Kanüle 14 gewährleistet. Gleichzeitig bietet die große Haftfläche einen erhöhten Schutz gegen Eindringen pathogener Stoffe in den Innenraum der Grundkörpers 4'.

Grundsätzlich genügt es aber, den Haftstreifen 5 lediglich im Bereich des Überganges 4''' zwischen dem Grunakörper 4' und den Laschen 4'''' anzubringen. Die Laschen 4'''' dienen hierbei als Greiflaschen zum bequemen Auseinanderschwenken der beiden Fortsatzhälften. Im übrigen wird bei enger Anpassung der als Anlagefläche 4'' ausgebildeten Innenfläche des Grundkörpers 4' an die Außenmaße der eingeführten Sonde ohnehin ein Vordringen pathogener Stoffe zum Applikationsort, insbesondere zum Punktionsort verhindert.

Zum manuellen Lösen der Haftverbindung zwischen den Haftstreifen 5 ist im Inneren des Fortsatzes 4 ein aus diesem herausragender Trennfaden 3 angeordnet. Der Trennfaden ist hierbei längs des Längsschlitzes des Fortsatzes 4 geführt. Er besteht beispielsweise aus Nylon.

Für den Fall, daß die gesamte Fläche der Laschen 4'''' mit einem Haftstreifen 5 beschichtet wird, ist der Trennfaden 3 im Bereich der Übergangsstelle 4''' zwischen Grundkörper 4' und Lasche 4'''' geführt. Durch Hochziehen des Trennfadens 3 werden die in Wirkstellung aneinanderhaftenden beiden Haftstreifen 5 weich und bequem wieder voneinander getrennt, so daß die im Fortsatz 4 befindliche Sonde leicht entfernt werden kann.

Auch in dem Fall, daß keine Laschen 4'''' vorgesehen sind, — mit anderen Worten also der Fortsatz 4 anstelle der beiden Übergangsbereiche 4''' zwischen dem Grundkörper 4' und den Laschen 4'''' lediglich zwei freie, mit einem Haftstreifen beschichtete, Innenkanten hat, kann die Haftungverbindung mittels des genannten Trennfadens 3 bei Bedarf wieder gelöst werden.

Wie aus Vorstehendem ohne weiteres ersichtlich, verlaufen die letztgenannten Innenkanten der Fortsatzhälften bzw. die Übergangsbereich 4''' in Längsrichtung des Fortsatzes 4 bis zum äußeren Rand der Durchtrittsöffnung 12. Sie enden an diesem Rand an der gleichen Stelle, an der auch die freien Kanten 6 des Flächengebildes 1 enden. Diese Maßnahme ermöglicht ein besonders bequemes Unterschieben des erfindungsgemäßen Verbandes unter den aus der Haut herausragenden Teil der Sonde bzw. Kanüle 14.

Unter Berücksichtigung der Außendurchmesser handelsüblicher Katheter, Kanülen und dergleichen beträgt der Innendurchmesser des Grundkörpers 4' — je nach konkreter Sonde zwischen 0,3 und 5 mm, wobei lediglich auf die gängigen Maße zurückgegriffen wurde. Vorzugsweise hat der Fortsatz 4 für Katheter eine Länge von ca. 50 mm, für Kanülen dagegen eine Länge von ca. 20 mm.

Die Figuren 5 und 6 zeigen gegenüber dem offenen Zustand des Fortsatzes 4 gemäß Figur 4 und dem geschlossenen Zustand bzw. der Wirkstellung des Fortsatzes 4 gemäß Figur 7 den halb offenen (Figur 5) und halb geschlossenen (Figur 6) Zustand des Fortsatzes 4. Zusätzlich sind in den Figuren 5 und 6 noch eine Kanüle 14 und dessen Lumen 9 dargestellt. Zwar ist zur besseren Unterscheidung zwischen der Kanüle 14 und der Innenwandung 4'' des Grundkörpers 4' ein Luftspalt gezeichnet. Bei paßgegerechter Ausbildung der als Anlagefläche dienenden Innenfläche 4'' des Grundkörpers 4 an die Außenfläche der Sonde 14 haften jedoch die beiden genannten Flächen durch Adhäsion so fest aneinander, daß eine Kontamination der Punktionsstelle mittels pathogener Substanzen längs dieses Weges ausgeschlossen ist. Im übrigen zeigt Figur 7 besonders anschaulich die Lage des Trennfadens 3 in Wirkstellung.

Das in Figur 10 dargestellte Ausführungsbeispiel weist zusätzlich ein die Durchtrittsöffnung 12 umgebendes Vlies 11 auf, das bei lückenlosem Aneinanderfügen der beiden freien Kanten 6 bzw. der einen freien Kante 6 und des restlichen Teiles des Flächengebildes 1 einen durchgehenden Ring bildet. Da das Vlies 11 selber zwei frei Kanten aufweist, wird durch die hierdurch vergrößerte Fläche der freien Kanten 6 ein lückenloses Aneinanderfügen besonders erleichtert. Das Vlies 11 ist mit entzündungshemmenden oder antibiotischen Pharmaka oder einer Mischimprägnierung daraus präpariert. Das die Punktionsstelle ringförmig umgebende Vlies 11 dient der zusätzlichen Abschirmung der Punktionsstelle gegen pathogene Erreger.

Sämtliche Haftschichten sind, soweit sie für direkten Kontakt mit der Haut vorgesehen sind, als hautfreundliche Haftschichten ausgebildet. Im übrigen sind die Haftschichten mit einem sterilen Silikonpapier abgedeckt, das nicht nur eine Kontamination der Haftschichten mit pathogenen Substanzen, sondern auch ein Zusammenkleben der Haftschichten bei der Konfektionierung verhindert.

Das Silikonpapier wird in üblicher Weise erst unmittelbar vor dem Anbringen der Haftschichten auf der Haut entfernt.

Die Ausführungsbeispiele des erfindungsgemäßen medizinischen Verbandes werden bevorzugt wie folgt gehandhabt: Nachdem die Sonde, insbesondere also der Katheter, die Kanüle oder die Nadel gelegt sind, wird der Verband unter sterilen Bedingungen der Verpackung entnommen. Das Silikonpapier wird aus dem Fortsatz 4 entfernt, und die freie Kante bzw. die freien Kanten 6 bis an die Einstichstelle an die Sonde herangeführt. Dann wird die Sonde in den Fortsatz 4 hineingelegt. Danach werden die Haftstreifen 5 der Laschen 4'''' oder der freien Flächen 4'''' des Schlitzes mit dem Zeigefinger und Daumen · zusammengedrückt. Danach wird das Silikonpapier von der Unterseite der Haftschicht 7 der Lasche 2 der Folie abgezogen und danach fest auf die Oberseite des Flächengebildes 1 bzw. der Folie angedrückt. Danach wird das Silikonpapier von der Haftschicht 10 auf der Unterseite des Verbandes, gegebenenfalls unter leichtem Anheben desselben, abgezogen. Vorzugsweise besteht das Silikonpapier aus zwei Hälften, so daß es nach links und nach rechts — gesehen in Richtung der Sonde — weggezogen werden kann. Hierbei legt sich der Verband an die Haut an und wird von oben fest angedrückt. Soll der Verband wieder entfernt werden, dann wird zunächst die Lasche 2 angehoben. Mit dem Trennfaden 3 werden die Haftstreifen 5 wieder voneinander getrennt. Danach wird die Sonde dem Fortsatz 4 entnommen und der Verband von der Haut abgezogen.

## Patentansprüche

1. Medizinischer Verband zur Fixierung einer durch die Haut eines Lebewesens in dessen Körper eingeführten Sonde (Nadel, Kanüle Katheter oder dgl.) (14) mit

a) einem an der Haut und am aus der Haut herausragenden Teil der Sonde (14) gleichzeitig befestigbaren Verbandsabschnitt (1, 4), der

b) ein zur Anlage an der Haut ausgelegtes Flächengebilde (1) und

c) einen mit dem Flächengebilde (1) verbundenen, von diesem stutzenartig vorstehenden und rohrförmig ausgebildeten Fortsatz (4) aufweist,

c. 1) dessen Innenwandung als Anlagefläche (4'') für den aus der Haut herausragenden Teil der Sonde (14) ausgeformt ist, und

c. 2) der — zum Zwecke einer manuellen Erweiterung des Fortsatzinnenraumes beim Einlegen oder Entfernen der Sonde (14) in den oder aus dem Fortsatz (4) — einen im wesentlichen achsparallel geführten und sich über seine gesamte Länge erstreckenden Schlitz aufweist, dadurch gekennzeichnet, daß

d) die beiden einander gegenüberliegenden Flächen (4'''') des Schlitzes eine selbsthaftende Schicht (5) aufweisen und

e) der Fortsatz (4) — zum manuellen Lösen der Haftverbindung der Flächen (4''') des Schlitzes — mit einem im Inneren des Fortsatzes (4) längs der Flächen (4''') des Schlitzes geführten und aus dem Fortsatz (4) herausragenden Trennfaden (3) bestückt ist.

2. Medizinischer Verband nach Anspruch 1, dadurch gekennzeichnet, daß der Fortsatz (4) über eine Verbindungsrippe mit dem Flächengebilde (1) verbunden ist.

3. Medizinischer Verband nach Anspruch 1 oder 2, dadurch gekennzeichnet, daß die Flächen (4''') des Schlitzes als Laschen (4'''') ausgebildet sind.

4. Medizinischer Verband nach wenigstens einem der vorstehenden Ansprüche, dadurch gekennzeichnet, daß der Fußbereich des Fortsatzes (4) allseitig vom Flächengebilde (1) umgeben ist und das Flächengebilde (1) in diesem Bereich eine Durchtrittsöffnung (12) aufweist.

5. Medizinischer Verband nach Anspruch 4, dadurch gekennzeichnet, daß

a) das Flächengebilde (1) wenigstens eine vom Rand der Durchtrittsöffnung (12) ausgehende, wenigstens bis in den Bereich der äußeren Umgrenzungskante des Flächengebildes (1) führende freie Kante (6) aufweist, die in derselben Ebene liegt, wie die Achse des Fortsatzes (4), und

b) die Innenkante des Schlitzes und die eine freie Kante (6) des Flächengebildes (1) jeweils im wesentlichen im selben Punkt des äußeren Randes der Durchgangsöffnung (12) enden.

6. Medizinischer Verband nach Anspruch 4, dadurch gekennzeichnet, daß, das Flächengebilde (1) eine seine eine freie Kante (6) überlappende Lasche (2) aufweist.

7. Medizinischer Verband nach wenigstens einem der vorstehenden Ansprüche, dadurch gekennzeichnet, daß der Fortsatz (4) aus demselben Material wie das Flächengebilde (1) besteht.

8. Medizinischer Verband nach wenigstens einem der vorstehenden Ansprüche, dadurch gekennzeichnet, daß der Fortsatz (4) und das Flächengebilde (1) aus einem für Röntgenstrahlen, sichtbares Licht und/oder Ultraschallwellen durchlässigen Material bestehen.

9. Medizinischer Verband nach wenigstens einem der vorstehenden Ansprüche, dadurch gekennzeichnet, daß der Fortsatz (4) und das Flächengebilde (1) elastisch sind und insbesondere aus Silikon-Kautschuk, Latex-Kautschuk, Polyurethan oder Polytetrafluoräthylen bestehen.

10. Medizinischer Verband nach wenigstens einem der vorstehenden Ansprüche, dadurch gekennzeichnet, daß das Flächengebilde (1) auf seiner dem Fortsatz (4) abgewandten Fläche eine an der Haut (20) selbsthaftende Haftschicht (10) aufweist.

11. Medizinischer Verband nach wenigstens einem der vorstehenden Ansprüche, dadurch gekennzeichnet, daß das Flächengebilde (1) auf seiner dem Fortsatz (4) abgewandten Fläche ein längs einer geschlossenen Bahn um die Durchtrittsöffnung (12) geführtes und mit Antibiotika, entzündungshemmenden Pharmaka oder einer daraus hergestellten Mischimprägnierung präpariertes Vlies (11) aufweist.


**Claims**

1. Medical dressing for the immobilisation of a tube (needle, cannula, catheter or the like) (14) introduced through the skin of an organism into its body, which dressing has

a) a section (1, 4) of the dressing which can be simultaneously fixed onto the skin and onto the part of the tube (14) which projects out of the skin, and which has

b) a sheet-like structure (1) designed to be laid on the skin and

c) a continuation (4) which is connected to the sheet-like structure (1) and projects like a nozzle from the latter and is designed in the form of a tube,

c. 1) the inner wall of which continuation being moulded as a support surface (4") for the part of the tube (14) which projects from the skin, and

c. 2) which — for the purpose of manual widening of the interior chamber of the continuation when the tube (14) is introduced into or removed from the continuation (4) — has a slit which is essentially parallel to the axis and extends over its entire length,
characterised in that

d) the two opposing surfaces (4"') of the slit have a self-adhesive layer (5) and

e) the continuation (4) is equipped — for manual release of the adhesive connection of the surfaces (4"') of the slit — with a separating thread (3) which runs inside the continuation (4) along the surfaces (4"') of the slit and projects out of the continuation (4).

2. Medical dressing according to Claim 1, characterised in that the continuation (4) is connected to the sheet-like structure (1) via a connection rib.

3. Medical dressing according to Claim 1 or 2, characterised in that the surfaces (4"') of the slit are designed as flaps (4"").

4. Medical dressing according to at least one of the preceding claims, characterised in that the base region of the continuation (4) is surrounded on all sides by the sheet-like structure (1), and the sheet like structure (1) has an opening (12) in this region.

5. Medical dressing according to Claim 4, characterised in that

a) the sheet-like structure (1) has at least one free edge (6) which starts from the rim of the opening (12), runs at least as far as the region of the outer limiting edge of the sheet-like structure (1), and is in the same plane as the axis of the continuation (4),

b) the inner edge of the slit and the one free edge (6) of the sheet-like structure (1) each terminate at essentially the same point on the outer rim of the opening (12).

6. Medical dressing according to Claim 4, characterised in that the sheet-like structure (1) has a flap (2) which overlaps its one free edge (6).

7. Medical dressing according to at least one of the preceding claims, characterised in that the continuation (4) is composed of the same material as that of the sheet-like structure (1).

8. Medical dressing according to at least one of the preceding claims, characterised in that the continuation (4) and the sheet-like structure (1) are composed of a material which is permeable to X-radiation, visible light and/or ultrasonic waves.

9. Medical dressing according to at least one of the preceding claims, characterised in that the continuation (4) and the sheet-like structure (1) are elastic and are composed of, in particular, silicone rubber, latex rubber, polyurethane or polytetrafluoroethylene.

10. Medical dressing according to at least one of the preceding claims, characterised in that the sheet-like strucutre (1) has, on its surface facing away from the continuation (4), an adhesive layer (10) which itself adheres to the skin (20).

11. Medical dressing according to at least one of the preceding claims, characterised in that the sheet-like structure (1) has, on its surface facing away from the continuation (4), a bonded web (11) which runs along a closed path around the opening (12) and is prepared with antibiotics, anti-inflammatory drugs or a mixed impregnation prepared therefrom.


**Revendications**

1. Pansement médical pour la fixation d'une sonde (aiguille, canule, cathéter ou analogue) (14) introduite à travers la peau d'un être vivant dans le corps de celui-ci, avec

a) une portion de pansement (1,4) pouvant être fixée simultanément à la peau et à la partie de la sonde (14) dépassant en saillie hors de la peau, et laquelle portion comporte

b) une structure à surface (1) conçue pour application contre la peau et

c) un prolongement (4) relié à la structure à surface (1) et dépassant en saillie en forme de manchon de celle-ci et réalisé sous forme tubulaire,

c.1) dont la paroi interne est conformé en tant que face d'appui (4") pour la partie de la sonde (14) dépassant de la peau, et

c.2) lequel comporte, dans le but d'un élargissement manuel de l'espace intérieur du prolongement lors de l'introduction de la sonde (14) dans le prolongement (4) ou de son enlèvement de celui-ci, une fente menée sensiblement parallèlement à l'axe et s'étendant sur sa longueur entière, caractérisé en ce que

d) les deux faces (4'''), mutuellement opposées, de la fente présentent une couche autocollante (5) et

e) le prolongement (4) est garni, pour le détachement manuel de la liaison adhérente des faces (4''') de la fente, d'un fil séparateur (3) mené à l'intérieur du prolongement (4) le long des faces (4''') de la fente et dépassant du prolongement (4).

2. Pansement médical selon la revendication 1, caractérisé en ce que le prolongement (4) est relié à la structure à surface (1) par l'intermédiaire d'une nervure de liaison.

3. Pansement médical selon la revendication 1 ou 2, caractérisé en ce que les faces (4''') de la fente sont réalisées sous forme de languettes (4'''').

4. Pansement médical selon au moins l'une des revendications précédentes, caractérisé en ce que la zone de pied du prolongement (4) est entourée de tous côtés par la structure à surface (1) et la structure à surface (1) présente un orifice de passage traversant (12) dans cette région.

5. Pansement médical selon la revendication 4, caractérisé en ce que

a) la structure à surface (1) comporte au moins un bord libre (6) partant du bord de l'orifice de passage traversant (12) et s'étendant au moins jusqu'au voisinage du bord de limitation extérieur de la structure à surface (1) et lequel est situé dans le même plan que l'axe du prolongement (4), et

b) le bord intérieur de la fente et l'un des bords libres (6) de la structure à surface (1) se terminent respectivement sensiblement au même point du bord extérieur de l'orifice de passage traversant (12).

6. Pansement médical selon la revendication 4, caractérisé en ce que la structure à surface (1) comporte une patte (2) recouvrant l'un de ses bords libres (6).

7. Pansement médical selon au moins l'une des revendications précédentes, caractérisé en ce que le prolongement (4) consiste en la même matière que la structure à surface (1).

8. Pansement médical selon au moins l'une des revendications précédentes, caractérisé en ce que le prolongement (4) et la structure à surface (1) consistent en une matière perméable aux rayons X, à la lumière visible et/ou à des ondes ultrasonores.

9. Pansement médical selon au moins l'une des revendications précédentes, caractérisé en ce que le prolongement (4) et la structure à surface (1) sont élastiques et consistent en particulier en caoutchouc siliconé, en caoutchouc au latex, en polyuréthane ou en polytétrafluoroéthylène.

10. Pansement médical selon au moins l'une des revendications précédentes, caractérisé en ce que la structure à surface (1) comporte, sur sa face tournée du côté opposé au prolongement (4), une couche adhésive (10) collant d'elle-même à la peau (20).

11. Pansement médical selon au moins l'une des revendications précédentes, caractérisé en ce que la structure à surface (1) comporte, sur sa face orientée du côté opposé au prolongement (4), une toison (11) s'étendant le long d'un trajet fermé autour de l'orifice de passage traversant (12) et préparé avec des antibiotiques, des médicaments antiphlogistiques ou une imprégnation mixte réalisée à partir de ceux-ci.

## Fig. 1 a

## Fig. 2 a

## Fig. 3 a

Fig. 1 b

Fig. 2 b

Fig. 3 b

Fig.4

Fig.5

Fig.6

Fig.7

Fig. 8 a

Fig. 9 a

Fig. 8b

Fig. 9b

Fig.10